# EUROPEAN PATENT SPECIFICATION

(11) **EP 3 447 488 B1**
(45) Date of publication and mention of the grant of the patent: **18.05.2022**
(21) Application number: 17188048.7
(22) Date of filing: 25.08.2017
(51) Int. Cl.: G01N 33/02, G01N 31/22, G01N 33/14, G01N 33/52, G01N 21/78

(54) **RAPID ASSAY FOR TOTAL PHENOL AND POLYPHENOL CONCENTRATION IN LIQUID FOOD SAMPLES**
SCHNELLTEST FÜR EINE GESAMT PHENOL- UND POLYPHENOLKONZENTRATION IN FLÜSSIGEN LEBENSMITTELPROBEN
ANALYSE RAPIDE DE CONCENTRATION TOTALE DE POLYPHÉNOL ET DE PHÉNOL DANS DES ÉCHANTILLONS D'ALIMENTS LIQUIDES

(43) Date of publication of application: 27.02.2019
(73) Proprietor: Smart Berries Sp. z o.o., 06-100 Pultusk (PL)
(72) Inventor: MAKAROVA, Katerina, 02-446 Warszawa (PL); ZAWADA, Katarzyna, 03-536 Warszawa (PL)
(74) Representative: Patpol Kancelaria Patentowa Sp. z o.o.

(56) References cited:
- CN-A- 106 353 312
- US-A1- 2008 026 470
- US-A1- 2013 034 908
- US-A1- 2014 162 368

## Description

### FIELD OF THE INVENTION

The invention is related to a rapid and easy estimation of phenol and polyphenol concentration in liquid food products, such as raw fruit juice, juice-containing beverages, green tea, black tea, yerba mate, coffee, red wine, and beer. Due to their biological origin, these liquids contain complex mixtures of numerous phenolic and polyphenolic compounds. Total phenol and polyphenol level is a characteristic relevant to anti-oxidative effect of these liquids that is believed to provide potential health benefits.

### BACKGROUND OF THE INVENTION

Polyphenols have been a subject of extensive research because of their pro-health activity. They are present in a variety of fruit, especially dark ones, vegetables, nuts, seeds, flowers, bark, beverages, and even some manufactured food due to the presence of the natural ingredients as components. They have been reported to exhibit anti-carcinogenic, anti-atherogenic, anti-ulcer, anti-thrombotic, anti-inflammatory, immunomodulating, antimicrobial, vasodilatory and analgesic effects. Interest in the research of polyphenols from various natural sources has grown because polyphenols can be utilized as antioxidants or colourants in the food industry, and they benefit human health in various ways. The beneficial effects of polyphenols on human health could be due to their free radical scavenger properties. Their high consumption has been postulated to be linked to a lower incidence of cardiovascular diseases. The Google Scholar search of the phrase "total polyphenols" shows over 900 publications in the first 5 months of 2017 and over 2000 in 2016, and for "polyphenols" - over 4000 and 20000 publications in the first 5 months of 2017 and in 2016, respectively. In PubMed, in 2017 there was over 750 hits for "polyphenols" in the first 5 months of 2017, and 1837 in 2016.

There are conferences organised on this subject (e.g., "World Congress on Polyphenols Applications"). There is also a database concerning the typical polyphenol content in various types of food products ("Phenol-Explorer", http://www.phenol-explorer.eu), and a USDA database concerning one group of polyphenols, namely flavonoids (USDA Database for the Flavonoid Content of Selected Foods, available at: https://www.ars.usda.gov/northeast-area/beltsville- md/beltsville- human- nutrition-research-center/nutrient-data-laboratory/docs/usda-special-interest-databases-on-flavonoids/). Generally, the more polyphenols in food, the better, but in case of some health conditions (e.g., cancer) their amount should be more or less strictly controlled.

Some methods for quantification of total polyphenol content are based on colorimetric measurements. Total phenols (or antioxidant effect) can be measured using the Folin-Ciocalteu reaction. Results are typically expressed as gallic acid equivalents (GAE).

The Folin-Ciocalteu method is now the most popular screening test for polyphenol content (over 1900 hits in Google Scholar (without citations) and 49 in PubMed in the first 5 months of 2017; as for PubMed database it should be stressed that it searches only through titles and abstracts of articles, and this method is usually described in "Materials and Methods" section of publications). The reagent contains phosphomolybdic and phosphotungstic acid complexes. The method is based on the transfer of electrons from phenolic compounds to form a blue chromophore constituted by a phosphotungstate/phosphomolybdate complex where the maximum absorption (measured at the range of 690-790 nm in alkaline environment) depends on the concentration of phenolic compounds. The reaction temperature has been used to reduce the time necessary to attain the maximum colour (T = 37°C). Generally, gallic acid is used as the reference standard compound and results are expressed as gallic acid equivalents. It should be kept in mind that this test can overestimate the amount of polyphenols due to the interference of other reducing substances like Vitamin C, ascorbate, or reducing sugars. However, the ratio of these compounds to polyphenols is usually close to constant in a given type of fruit.

On March 6, 2017 AOAC INTERNATIONAL invited method authors and developers to submit relevant methods that may meet the AOAC Standard Method Performance Requirements^{®} (SMPR^{®}) for the Methods for the Estimation of Total Phenolic Content Using the Folin-Ciocalteu Assay (SMPR 2015.009). All submitted methods are now subjected to evaluation by an AOAC Expert Review Panel, who review them for potential AOAC First Action Official Methods^{SM} status. Any resulting approved/adopted Official Methods of Analysis may be used as a reference method. However, any official method would still require laboratory equipment and sample preparation (at least sample dilution in case of analysis of juice, such as berry juice), and thus it would be unsuitable for use in field conditions or at home by an average end user.

The plant polyphenols measurements in laboratories are preferably performed by high-performance liquid chromatography (HPLC), as it gives information about both composition and concentration of polyphenols. However, the composition of polyphenols in a given type of fruit does not usually vary significantly, contrary to their total amount. Thus, once the composition of polyphenols in a given plant material has been determined with HPLC, other methods like spectrophotometric and titration ones are also used to determine the total content of polyphenols. They are usually faster and less costly than HPLC technique. However, most of these methods require laboratory equipment, preparation of reagents and skilled personnel to perform.

In the prior art there exists methods available to a wider group of end-users, which require neither specialised laboratory nor skills. One of such methods is based on a reaction of polyphenols with chromogen and is utilized in CDR Winelab system comprising small spectrophotometers with prefilled cuvettes. This system can be used in winery or fruit processing plant, however it still requires at least a power source and a table. Moreover, even though it is not necessary to employ a skilled laboratory technician to use said system, its user still needs some previous training regarding operation of the device and automatic pipettes. Furthermore, for samples with a high level of polyphenols, >6 g/L (for results expressed as gallic acid equivalents, GAE), an additional preliminary preparation of samples is required. Thus, this approach is not suitable to use in the field.

There are also portable spectrophotometers (such as HunterLab MiniScan line) that could be used for performing polyphenol tests in the field, but they require equipment calibration by the user with a reaction vessel used (they do not use standard spectrophotometric cuvettes), and completing the reagents for the reaction. Therefore, a skilled technician is needed to use them to determine polyphenol content.

The technical solution disclosed in patent application number CN105259169A ("Test paper for detecting tea polyphenol quickly and preparation method of test paper") and in patent application number CN 101294912 B ("Tea polyphenol detecting test paper, standard colour comparison card and usage thereof') is based on ferrous tartrate method. This reaction is practically specific to catechins, as other types of polyphenols react at a much smaller rate. The catechins are, by large, the main group of polyphenols present in tea, because almost all tea polyphenols are catechins and catechin polymers. However, in fruit the catechins are only one of the co-present polyphenol groups. Therefore, the tests disclosed in these publications are not appropriate for detection and quantification of polyphenols in fruit and fruit products, for example berries, because it would lead to underestimation of polyphenol content. Moreover, the semi-quantification of polyphenols is performed by a visual comparison with a standard colour card, which is highly subjective as the perception of colours varies greatly in the population. Furthermore, for dark-coloured fruit (like berries), the colour of juice interferes with the colour of a reaction product.

Another strip test disclosed by Dieter Tanzer and Uwe Kraetschmer (publication EP 1779105 A1 and US 2008/026470) was based on a paper test utilizing Folin-Ciocalteu method. However, this method is suitable for quantification of polyphenols in the range up to 0.3 g/L (GAE), which is well below the typical values for freshly squeezed high-polyphenol fruit juice or red wine. Therefore, there is a need for an additional preliminary preparation of samples. Again, the semi-quantification of polyphenols is performed by a visual comparison with a standard colour card and quantification with a laboratory equipment (a reflectometer).

CN 106353312 A discloses a method for determining the content of polyphenols in tea using of a microfluidic chip. In the said method a Folin-Ciocalteu reagent is used, however it requires an access to laboratory equipment (automatic pipettes) and laboratory skills, as the pipetting of a single microliters is required. A further drawback of this method is that a sample has to be diluted prior to the analysis. Moreover, this method requires use of gallic acid standard, which is unstable in solution. Finally, the colour analysis is based on visual comparison of the colour in the reaction field of the sample and of the standard solution(s), which could be compromised by the colour of a sample (e.g. berry juice), or has to be done using separate programs for colour analysis.

A method for detection of certain antioxidants, including polyphenols, which uses ceria nanoparticles is disclosed in US 2014/0162368. This method requires a series of dilutions prior to analysis, as the quantification of antioxidants is based on the slope of colour intensity versus sample concentration function. Thus, it is difficult to perform this method outside a laboratory. All colour analysis has to be done externally, as there is no integrated method of colour analysis, and no compensation for different light conditions and camera properties is provided.

US 2013/0034908 A1 discloses a use of a Folin-Ciocalteu reagent at a paper analytical device (PAD) for detection of phenolic impurities in pharmaceutical preparations. However, this application describes only the yes/no procedure, and the quantitative analysis would require a preparation of a calibration curve by the user. Thus laboratory skills and equipment would be required. Although in this method a smartphone camera could be used for capturing a picture, there is still a need for external device/software that would perform colour analysis.

Therefore, there still exist a need for a polyphenol detection method, which would allow for rapid estimation of total polyphenol content in a sample and, at the same time, would be easy to use and would not require any special preparation before.

### SUMMARY OF THE INVENTION

The invention provides a test method according to claim 1 for the determination of phenol and polyphenols concentration in liquid water based food (such as coffee, berry, juice, fruit smoothies or wine) with high concentration of polyphenols. In the method of the invention the paper test is used in combination with a smartphone used for the result interpretation. The test paper is prepared by soaking an absorbent material with Folin-Ciocalteu reagent, previously diluted with either deionized water or 1% xanthan gum solution. The dilution of the reagent is 10 - 20 fold.

The phenol and polyphenol content in liquid food can be quickly detected by placing a drop of juice on the test paper, and then adding a drop of sodium carbonate aqueous solution. After a minute of reaction, the results are interpreted by means of smartphone application based on the image recorded by smartphone camera. The test paper is suitable for quickly measuring the approximate polyphenol content in water-based liquid food products (such as squeezed berries and other fruit, juices, fruit smoothies, coffee, wine). The paper test in combination with a smartphone camera and mobile application has the advantages of a simple, convenient and rapid method, featuring low cost and easy implementation.

In an another aspect, the invention provides a test kit according to claim 13, comprising ready-to-use test papers, a dripping bottle containing aqueous sodium carbonate solution, a colour card, disposable pipettes, and optionally plastic bags useful for sampling.

### BRIEF DESCRIPTION OF THE DRAWINGS

Fig. 1 is a schematic representation of test paper placed on a colour card.
Fig. 2 presents calibration curves (colour value vs. GAE) for blue (a) and green (b) colours obtained in Example 1.
Fig. 3 is a photograph of a sample of Example 2 (sweet cherry 1) on a test paper placed on colour card before (a) and after (b) application of sodium carbonate solution.
Fig. 4 is a photograph of a sample of Example 3 (sweet cherry 2) on a test paper placed on colour card before (a) and after (b) application of sodium carbonate solution.
Fig. 5 presents photographs of a sample of Example 4 (chokeberry) on a test paper placed on colour card after application of sodium carbonate solution original photograph (a) and split into RGB channels: red (b), green (c) and blue (d).
Fig. 6 presents a correlation plot for results obtained by the method of the invention and laboratory test results for different samples (Example 5).

### DETAILED DESCRIPTION OF THE INVENTION

### Definitions

Within this patent application, the test paper is generally understood to mean any acid-resistant absorbent material, such as hard filter paper, filter cloth, or non-woven fabric, that is impregnated with the diluted or undiluted Folin-Ciocalteu reagent.

The Folin-Ciocalteu reagent is a liquid acidic mixture of phosphomolybdate and phosphotungstate. It can be prepared according to the literature (V. L. Singleton et al., Methods in Enzymology, 1999, 299, 152-178, see page 155). This reagent is also readily available from commercial suppliers, e.g., Sigma-Aldrich and P.O.Ch.

Within this patent application, the term "smartphone" is generally understood as a mobile personal computer or a tablet that can run third-party software components and is equipped with a colour digital camera. It will be understood that for the purpose of the invention the smartphone can be substituted with a separate camera and any computer means capable of running an application for analysis of the photograph taken by a camera.

The colour card, also referred to the colour reference card, is a card of durable plastic or cardboard, with red, green, and blue (RGB) reference areas printed thereon. The RGB reference areas are used for colour normalization during colour value analysis. Thus phenol and polyphenol content reading does not depend on, for example, lighting conditions when a photograph of the sample is taken or camera characteristics.

### Method of the invention

The test method of the invention covers a range of from 1 to 10 g/L of gallic acid equivalents (GAE), thus enabling the direct assessment of phenol and polyphenol content in juice on the spot, e.g., in the field or when making a decision about buying a raw plant material. To avoid subjective visual comparison of colours with a standard colour card, the inventors propose to use a smartphone camera. This now widespread device enables the determination of a formed chromogen colour without the interferences from the sample colour. This is especially important in case of dark fruit, such as berries (e.g. chokeberries, blueberries and the like), which, at the same time, are the richest in polyphenols. Contrary to the previously described approaches, the test method of the invention does not require any additional equipment, as a smartphone is a widely used device. Furthermore, the implementation of mobile application into the test enables taking into account the presence of interfering compounds, e.g., vitamin C, ascorbate, or reducing sugars, and recalculation of the data with allowance for average ratio of polyphenols and interfering compounds. This is not possible with the use of only test strip or a photometer.

The liquid food materials to be sampled may be fruit pulp, raw fruit juice, fruit-juice containing beverages, tea (e.g., black and green tea), yerba mate, coffee, red wine, beer and other coloured liquids containing phenols and polyphenols of natural origin.

### Materials

The Folin-Ciocalteu reagent may be prepared by mixing, boiling and refluxing at least sodium tungstate, sodium molybdate, orthophosphoric acid and hydrochloric acid with a subsequent addition of lithium sulphate and bromine according to the literature procedure (for details, see V. L. Singleton et al., Methods in Enzymology, 1999, 299, 152-178, page 155).

The Folin-Ciocalteu reagent is diluted in a ratio of 1:10 to 1:20 by volume with deionised water or with a 1% xanthan gum in deionised water.

The test paper may be prepared, either on the spot or in advance, by impregnating an absorbent material with the above-mentioned diluted Folin-Ciocalteu reagent. The test paper may be also prepared by applying a liquid layer of the undiluted Folin-Ciocalteu reagent to an absorbent material with a press. The test paper may be then dried, and stored until use.

Either the bottom side or both sides of the test paper may be protected by a protective layer of impermeable polymer such as plastic or chitosan. A window for a drop of sample should be allowed in the front-side protective layer if both sides of the test paper are protected.

The alkaline solution is a solution of 20 wt. % sodium carbonate in deionised water. Preferably, it is contained in a dripping bottle, to be conveniently dosed.

### Equipment

In general, any smartphone model can be used if it can run third-party software components and is equipped with a colour digital camera. In the examples specified below, an Asus Zenfone GO was used, running under Android 5.1 operating system.

### Measurement

The test may be carried out at ambient temperature. In general a drop of the sample is put onto the test paper, and a drop of the alkaline solution is put at the same place on the test paper. The sample is a liquid sample, such as fruit juice (e.g., juice freshly squeezed from fruits or commercially available), fruit products (e.g. fruit smoothies), wine, brewed coffee or tea. It is important that any particles of fruit present on the test paper, are removed before analysis, as the particles might interfere with a colour interpretation.

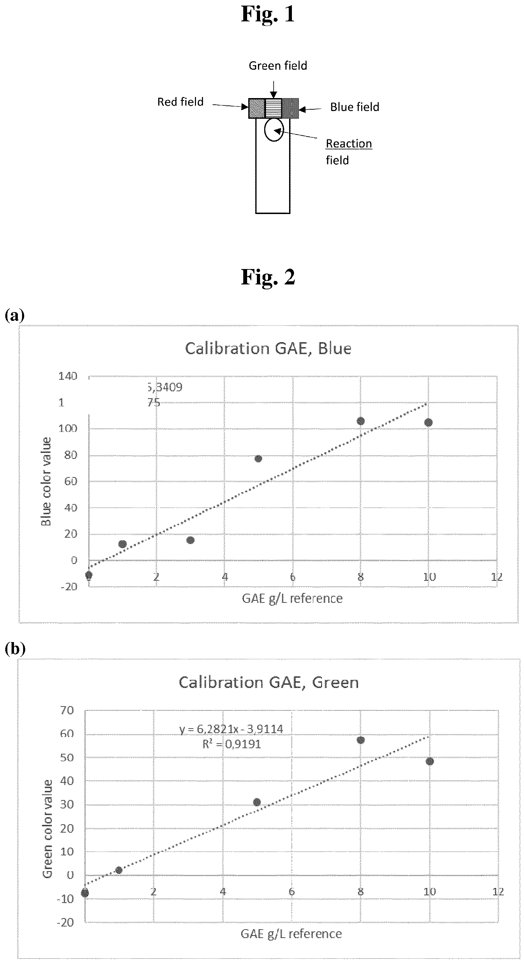

After 1 to 3, 4, 5 or 6 minutes, the test paper is placed on the colour card so that the reaction area is next to the RGB colour areas. Fig. 1 presents a schematic representation of the test paper placed on a colour card. Then a photo of the test spot along with the RGB areas is taken, and the image is analysed using a dedicated smartphone application. This analysis is also referred herein as the analysis in the RGB scheme. As a result, the approximate concentration of total polyphenols is obtained.

Instead of performing the analysis using the smartphone application, the photo may be sent for analysis to the remote computing device.

### Principle of analysis

The location of the test spot and the RGB areas are first identified in the obtained photo. Then the colours are converted to the standard RGB scheme (8 bit colour coding for each colour channel, intensity range of 0 - 255) and each colour channel is averaged. During analysis the colour is normalized with respect to RGB areas on the colour reference card. Then the colours are compared with a standard curve obtained by calibration using known concentrations of gallic acid, and the gallic acid equivalent (GAE, in g/L) is calculated.

In general, the blue channel component is taken into account, since its intensity closely corresponds to the colour complex being the product of the Folin-Ciocalteu reaction. Unexpectedly, the intrinsic colour of fruit juice, that makes visual estimation practically impossible, does not interfere with the above-described method of analysis, if the blue channel component of the test spot is analysed.

There are two types of samples that can be analysed by the method of the invention:
1) the first one with weak colours (that do not interfere with the interpretation of the colour from a polyphenols reaction with a reagent in a test paper); and
2) the second one with strong colours (some cherries, strawberries, smoothies etc), which disturb the analysis and lead to over estimation of the results.

That is why an additional step of colour interpretation is introduced into the method of the invention: the colour analysis of the sample drop before sodium carbonate solution is added. Therefore, the picture of a test paper has to be taken twice - before and after the addition of sodium carbonate solution. The photo taken before the addition of the sodium carbonate solution undergoes the colour analysis and the blue colour value is determined. If the natural sample colour is not disturbing the range of polyphenol content (i.e. the obtained value for the first picture is 0), the analysis is performed with respect to the blue colour calibration curve and the picture taken after the addition of the sodium carbonate solution is analysed with respect to the blue colour value, based on which the polyphenol content in the sample is determined.

If the natural colour sample is disturbing the range of polyphenol content (i.e. the obtained value for the first picture is >0), the analysis is performed with respect to the green colour calibration curve. The difference between green colour after the addition of the sodium carbonate solution and green colour before the addition of the sodium carbonate solution is analysed.

### Uses

The method of the invention is useful for determination of total phenol and polyphenol concentration in aqueous liquids, especially for the liquid food samples, having high polyphenol concentrations (1- 10 g/L GAE), with no need for dilution of the sample, and regardless of the sample colour.

The inventive method is particularly suitable for:
- evaluation of fruit material prior to purchase;
- determining the proper moment for fruit picking;
- monitoring the fruit processing (for food technologists in fruit processing plants)
- checking the quality of homemade or commercially available fruit products by consumers.

### EXAMPLES

### Example 1 Calibration curves

The calibration curves were obtained based on ethanol solution of gallic acid (a reference compound) having concentration in the range 0 to 10 g/L. For each concentration of the reference compound a colour analysis is performed as described above. In short, a drop of standard solution is placed in the reaction filed of the test paper, i.e. the paper impregnated with diluted Folin-Ciocalteu reagent as described above in *"Materials"* section of the present description. Next, a drop of the alkaline solution (i.e. 20 wt. % sodium carbonate in deionised water) is put at the same place on the test paper. The test paper is placed on a colour reference card and after 4, 5 and 6 minutes of the reaction the photographs are taken with a smartphone and each of the images is analysed in the RGB scheme using the smartphone application, wherein the colour is normalized in accordance with the colour reference card.

The blue and green colour values are recorded for each reference compound concentration and the average values are plotted against the reference compound concentration resulting in calibration curves for BLUE and GREEN colour in RGB. The representative calibration curves blue (a) and green (b) colour are presented in Fig. 2.

### Example 2 Polyphenol content in sweet cherry 1 - weak colour sample

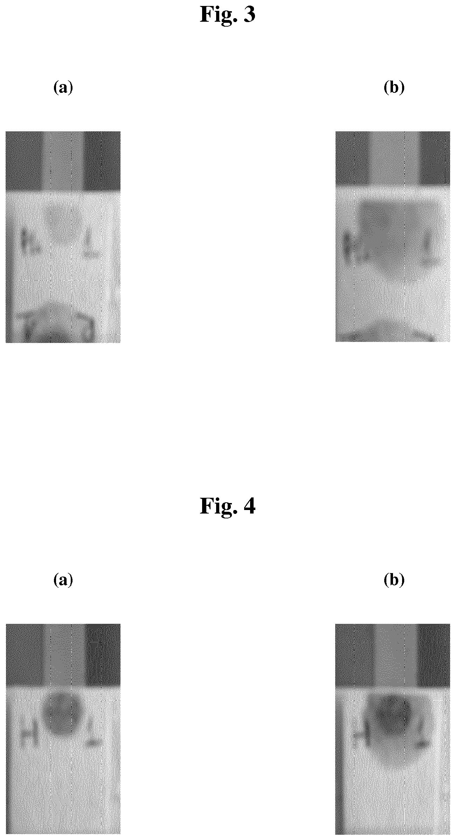

A drop of sweet cherry juice is placed in the reaction filed of the test paper as described for a reference compound solution in Example 1. Before the alkaline solution is added, a photograph of a sample on the test paper on the colour reference card is taken as presented in Fig. 3a. The sample image undergoes a colour analysis in RGB scheme and the blue colour value is calculated for the sample. The blue colour value obtained for the image indicates that the polyphenol content detected before reaction equals 0 (i.e. the blue colour value is not disturbing the range of polyphenol content). A drop of the alkaline solution described in Example 1 is placed over the sample on the test paper, and the photographs are taken 4, 5 and 6 minutes of the reaction. An exemplary image of the sample after alkaline solution is added is presented in Fig. 3b. Each of the images is analysed in RGB scheme using the smartphone application and a blue colour value is determined. In reference to blue colour calibration curve the amount of polyphenols is determined.

Table 1, below, presents colour values recorded for sample before alkaline solution is applied, as well as for samples after 4, 5 and 6 minutes after reaction is initiated. In the last column of the table the determined amount of polyphenols is presented as g GAE /L.

**Table 1**

| | Minutes after the addition of the sodium carbonate solution | Blue | Red | Green | Results obtained by the method of the invention, rounded to integer numbers |
|---|---|---|---|---|---|
| colour before addition of the sodium carbonate solution | - | 9.405 | -1.041 | -7.479 | 0 |
| colour after addition of the sodium carbonate solution | 4 | 23.233 | 45.524 | 22.591 | 1 |
| | 5 | 22.474 | 46.867 | 20.83 | 1 |
| | 6 | 23.386 | 49.467 | 24.865 | 1 |
| | **average** | **23.031** | **47.286** | **22.762** | **1** |

The same sample was analysed using a spectrophotometric Folin-Ciocalteu assay and the polyphenol content determined amounted to 1.355 g GAE/L. This shows good correlation of the results obtained by the method of the invention with the results of the spectrophotometric Folin-Ciocalteu assay.

### Example 3 Polyphenol content in sweet cherry 2 - strong colour sample

A drop of sweet cherry juice is placed in the reaction filed of the test paper as described in Example 2. Before the alkaline solution is added, a photograph of a sample on the test paper on the colour reference card is taken as presented in Fig. 4a. The sample image undergoes a colour analysis in RGB scheme and the blue colour value is calculated for the sample. The blue colour value obtained for the image indicates that the polyphenol content detected before reaction equals 4 (i.e. the blue colour value obtained would influence the reading for the polyphenol content). The analysis proceeds as described in Example 2, i.e. a drop of the alkaline solution is added and the photographs are taken 4, 5 and 6 minutes of the reaction. However, for polyphenol content analysis, the application refers to green colour calibration curve and also utilizes the differences in green colour values recorded for the sample before and after the alkaline solution is added.

Table 2, below, presents colour values recorded for sample before alkaline solution is applied, as well as for samples after 4, 5 and 6 minutes after reaction is initiated. In the last column of the table the determined amount of polyphenols is presented as g GAE /L.

**Table 2**

| | Minutes after the addition of the sodium carbonate solution | Blue | Red | Green | Results calculated by application, rounded to integer numbers, using the BLUE calibration curve | Results calculated by application, rounded to integer numbers, using the GREEN calibration curve | Results rounded to integer numbers calculated by application based on GREEN calibration curve and the difference between the values recorded for two pictures (before and after alkaline solution is added) |
|---|---|---|---|---|---|---|---|
| color before addition of the sodium carbonate solution | 0 | 59.773 | 25.694 | 38.938 | 4 | 7 | |
| color after addition of the sodium carbonate solution | 4 | 75.179 | 80.241 | 56.41 | 6 | 9 | 2 |
| | 5 | 74.413 | 83.646 | 63.086 | 6 | 10 | 3 |
| | 6 | 68.475 | 77.076 | 60.501 | 5 | 10 | 3 |
| | **average** | **72.69** | **80.32** | **60.00** | **5.67** | **9.67** | 3 |

Therefore, the amount of polyphenols in the sample calculated using the method of the invention amounted to 3. The result of a spectrophotometric Folin-Ciocalteu assay analysis for the same sample was 2.885 g GAE/L, which shows good correlation between the two methods.

### Example 4 Polyphenol content in black chokeberry

Several black chokeberry (*Aronia melanocarpa*) fruit are put into a plastic bag and squeezed to obtain some juice. A drop of the juice is transferred from the bag to a test paper, as described in earler ecamples, using a single-use plastic pipette (70 drops per milliliter).

A drop of the alkaline solution is placed at the spot marked by the juice on the test paper from the dripping bottle. After 3 minutes, the test paper is placed on the colour reference card next to the RGB areas. Then a colour photo is taken and the image is analysed by the mobile application.

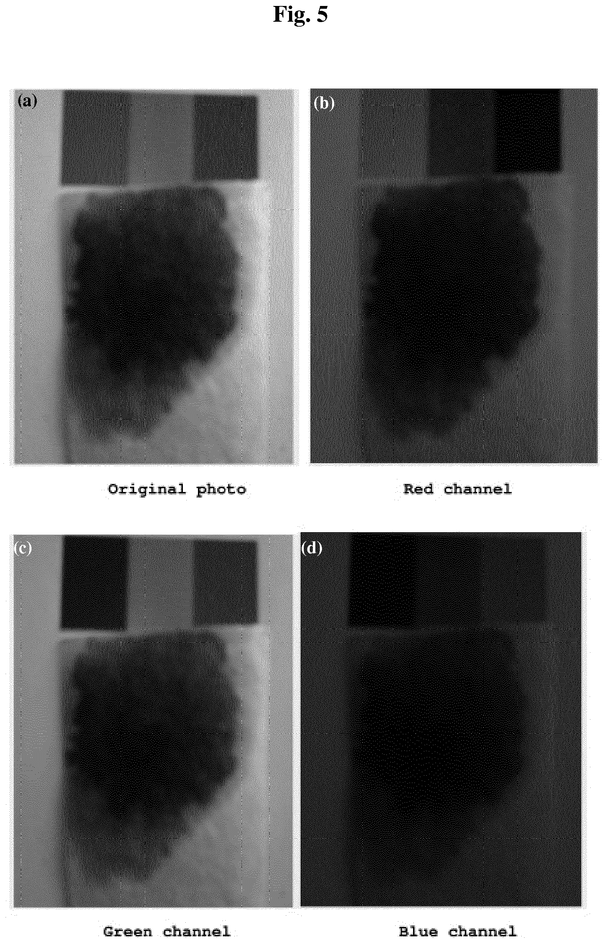

The analysis is illustrated in Fig. 5, where the original photograph (a), as well as the photograph split into RGB channels (b - d) are presented. The data is also summarised in Table 3 below.

**Table 3**

| Sample values | | | Reference values | | | Difference | | |
|---|---|---|---|---|---|---|---|---|
| Red | Green | Blue | Red | Green | Blue | Red | Green | Blue |
| 20.61 | 20.02 | 30.80 | 129.15 | 159.86 | 143.58 | 108.54 | 139.66 | 112.78 |

The smartphone application recalculates the results of analysis to obtain the concentration of total phenols and polyphenols present in the sample (in reference to the calibration curves).

For this sample, the total contents of polyphenols in black chokeberry juice was found to be 7.0 g/L of gallic acid equivalents.

### Example 5 Analysis of other samples and result correlation

As described above the corresponding analysis was performed for many different products. Table 4 below presents data for some of the samples obtained using the method of the invention (SmartBerriese test). For comparison the results of a spectrophotometric Folin-Ciocalteu assay analysis for the same sample were also presented.

**Table 4**

| | Sample | SmartBerries test GAE [g/L] | Spectrophotometric Folin-Ciocalteu assay GAE [g/L] |
|---|---|---|---|
| **Fruit** | | | |
| Frozen | Strawberry 1 | | 1.37 |
| Frozen | Banana 1 | | 0.25 |
| Frozen | Papaya 1 | | 0.43 |
| Frozen | Strawberry 2 | | 1.40 |
| Frozen | Mango 2 | | 0.25 |
| Frozen | Pineapple 2 | | 0.58 |
| Fresh | Raspberry | | 3.35 |
| Fresh | Sweet cherry 1 | | 2.89 |
| Fresh | Sweet cherry 2 | | 1.36 |

| **Juice** | | | |
|---|---|---|---|
| | Cherry 1 | | 3.64 |
| | Cherry 2 | | 3.16 |
| | Pomegranate, Vital Fresh | | 2.80 |
| | Black chokeberry | | 7.00 |
| | Apple + raspberry 1 | | 0.66 |
| | Apple + raspberry 2 | | 0.56 |

| **Smoothies** | | | |
|---|---|---|---|
| "Zielone Wzgórza" banana, strawberry, papaya | 0 | 0.49 | |
| "Zielone Wzgórza" pineapple, stawberry, mango | 1 | 0.88 | |
| "Kocham Smoothies" "detoks" | 1 | 0.25 | |
| "Kocham Smoothies" "owocowa moc" | 3 | 2.39 | |
| "Kocham Smoothies" "tropikalne niebo" | 1 | 0.44 | |

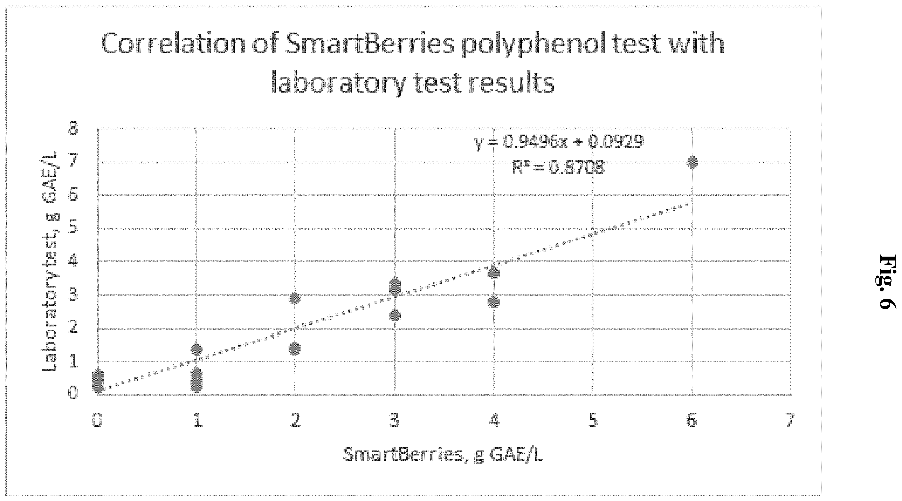

The results obtained using the assay method of the invention were plotted against the results obtained in Spectrophotometric Folin-Ciocalteu assay. The correlation of the results is presented in Fig. 6. The method of the invention, even though very simple in use shows good correlation with the results obtained using traditional spectrophotometric assay. This shows that the method of the invention can be successfully used for phenol and polyphenol content analysis.

## Claims

1. An assay method for analysis of total phenol and polyphenol concentration in aqueous liquid samples, comprising the steps of
(a) putting a drop of the sample onto the test paper previously impregnated with the Folin-Ciocalteu reagent thus forming a test spot;
(b) adding a drop of an aqueous alkaline solution to the test spot on the test paper;
(c) placing the test paper on the reference colour card with the test spot in proximity of red, green, and blue reference areas printed on the reference colour card;
(d) taking a colour photograph of the test spot on the test paper along with the red, green, and blue areas on the reference colour card;
(e) performing colour analysis of the photograph in the RGB scheme, normalized in reference to the red, green, and blue areas on the reference colour card, and obtaining colour value measurement;
(f) assessing of total phenol and polyphenol concentration based on red, green and blue, preferably green and blue, colour value measurements.

2. The assay method claim 1, wherein after step (a) of putting a drop of the sample onto the test paper and before step (b) of adding an aqueous alkaline solution to the test spot, the following steps are performed:
(aa) placing the test paper on the reference colour card with the test spot in proximity of red, green, and blue reference areas printed on the reference colour card;
(ab) taking a colour photograph of the test spot on the test paper along with the red, green, and blue areas on the reference colour card;
(ac) performing colour analysis of the photograph in the RGB scheme normalized in reference to the red, green, and blue areas on the reference colour card and determining the blue colour value of the sample, wherein if said blue colour value does not interfere with the range of polyphenol content, said polyphenol content is estimated based on blue colour value, otherwise it is estimated based on the green colour value obtained as a difference of green colour values for photographs taken in step (ab) and (d).

3. The assay method of claim 1 or 2, wherein the analysis in step (ac) and (e) is carried out using computer means capable of running an application for analysis of the photograph in the RGB scheme.

4. The assay method of any claims 1 - 3, wherein the photographs are taken and the colour analyses of said photographs are carried out using the same device.

5. The assay method of claim 4, wherein the device is a smartphone.

6. The assay method of any claims 1 - 5, wherein the sample is selected from raw fruit juice, juice-containing beverages, green tea, black tea, yerba mate, coffee, red wine, and beer.

7. The assay method of claim 6, wherein the sample is freshly squeezed raw fruit juice.

8. The assay method of any claim 1 - 7, wherein the photograph in step (d) is taken 1 - 7 minutes after step (b) is performed.

9. The assay method of any claim 1 - 8, wherein the aqueous alkaline solution from step (b) is sodium carbonate solution.

10. The assay method of any claim 1 - 9, wherein the test paper is obtained by impregnating an acid-resistant absorbent material with the diluted or undiluted Folin-Ciocalteu reagent and dried.

11. The assay method of claim 10, wherein the acid-resistant absorbent material is selected from a group comprising hard filter paper, filter cloth, or non-woven fabric.

12. The assay method of claim 10 or 11, wherein the Folin-Ciocalteu reagent is diluted in 10-20 fold range with either deionized water or 1% xanthan gum solution.

13. A test kit for determination of total phenol and polyphenol concentration in aqueous liquids, comprising:
one or more test papers obtained by impregnating an acid-resistant absorbent material with the diluted or undiluted Folin-Ciocalteu reagent and dried;
one or more disposable pipettes;
sodium carbonate solution in deionised water, contained in a dropping bottle ;
**characterised in that** it further comprises a reference colour card, with red, green, and blue areas printed thereon.

14. The test kit of claim 13 that it further comprises one or more disposable plastic bags for squeezing out juice from fruits.

## Patentansprüche

1. Ein Testverfahren zur Analyse der gesamten Phenol- und Polyphenolkonzentration in wässrigen Flüssigkeitsproben, folgende Schritte umfassend:
(a) Auftragen eines Tropfens der Probe auf ein zuvor mit dem Folin-Ciocalteu-Reagenz imprägniertes Testpapier, wodurch ein Testfleck entsteht;
(b) Zugeben eines Tropfens einer wässrigen alkalischen Lösung auf den Testfleck auf dem Testpapier;
(c) Aufbringen des Testpapiers auf die Referenzfarbkarte, mit dem Testfleck in der Nähe der auf der Referenzfarbkarte aufgedruckten roten, grünen und blauen Referenzbereiche;
(d) Aufnehmen eines Farbbildes des Testflecks auf dem Testpapierstreifen, zusammen mit den roten, grünen und blauen Bereichen auf der Referenzfarbkarte;
(e) Durchführen einer Farbanalyse des Bildes nach dem RGB-Schema, normalisiert in Bezug auf die roten, grünen und blauen Bereiche auf der Referenzfarbkarte, sowie Ermittlung der Farbwertmessung;
(f) Bewerten der gesamten Phenol- und Polyphenolkonzentration auf der Grundlage von Messungen der roten, grünen und blauen, vorzugsweise grünen und blauen gemessenen Farbwerte.

2. Das Testverfahren nach Anspruch 1, wobei nach dem Schritt (a), bei dem ein Tropfen der Probe auf den Testpapierstreifen aufgetragen wird und vor dem Schritt (b), bei dem eine wässrige alkalische Lösung auf den Testfleck zugegeben wird, die folgenden Schritte durchgeführt werden:
(aa) Aufbringen des Testpapiers auf die Referenzfarbkarte, mit dem Testfleck in der Nähe der auf der Referenzfarbkarte aufgedruckten roten, grünen und blauen Referenzbereiche;
(ab) Aufnehmen eines Farbbildes des Testflecks auf dem Testpapierstreifen, zusammen mit den roten, grünen und blauen Bereichen auf der Referenzfarbkarte;
(ac) Durchführen einer Farbanalyse des Bildes nach dem RGB-Schema, normalisiert in Bezug auf die roten, grünen und blauen Bereiche auf der Referenzfarbkarte, und Ermitteln des blauen Farbwerts der Probe, wobei wenn der blaue Farbwert den Bereich des Polyphenolgehalts nicht beeinträchtigt, der Polyphenolgehalt auf der Grundlage des blauen Farbwerts geschätzt wird, andernfalls wird er auf der Grundlage des grünen Farbwerts geschätzt, der als eine Differenz der grünen Farbwerte für die im Schritt (ab) und (d) aufgenommenen Bilder erhalten wird.

3. Das Testverfahren nach Anspruch 1 oder 2, wobei die Auswertung im Schritt (ac) und (e) unter Verwendung von Computermitteln durchgeführt wird, die in der Lage sind, eine Anwendung zur Analyse des Bildes nach dem RGB-Schema auszuführen.

4. Das Testverfahren nach einem der Ansprüche 1 bis 3, wobei die Bilder mit demselben Gerät aufgenommen und die Farben auf den Bildern analysiert werden.

5. Das Testverfahren nach Anspruch 4, wobei das Gerät ein Smartphone ist.

6. Das Testverfahren nach einem der Ansprüche 1 bis 5, wobei die Probe aus rohem Fruchtsaft, safthaltigen Getränken, grünem Tee, schwarzem Tee, Yerba Mate, Kaffee, Rotwein und Bier ausgewählt wird.

7. Das Testverfahren nach Anspruch 6, wobei die Probe ein frisch gepresster roher Fruchtsaft ist.

8. Das Testverfahren nach einem der Ansprüche 1 bis 7, wobei das Bild im Schritt (d) 1 bis 7 Minuten nach dem Schritt (b) aufgenommen wird.

9. Das Testverfahren nach einem der Ansprüche 1 bis 8, wobei die wässrige alkalische Lösung aus dem Schritt (b) eine Natriumcarbonat-Lösung ist.

10. Das Testverfahren nach einem der Ansprüche 1 bis 9, wobei das Testpapier durch Imprägnation eines säurebeständigen absorbierenden Stoffes mit dem verdünnten oder unverdünnten Folin-Ciocalteu-Reagenz und Trocknung erhalten wird.

11. Das Testverfahren nach Anspruch 10, wobei das säurebeständige absorbierende Stoff aus einer Gruppe ausgewählt ist, die hartes Filterpapier, Filtertuch oder Vliesstoff umfasst.

12. Das Testverfahren nach Anspruch 10 oder 11, wobei das Folin-Ciocalteu-Reagenz entweder mit entionisiertem Wasser oder mit einer 1 %igen Xanthangummi-Lösung im 10-20fachen Bereich verdünnt wird.

13. Ein Testsatz zur Bestimmung der gesamten Phenol- und Polyphenolkonzentration in wässrigen Flüssigkeiten, umfassend:
ein oder mehrere Testpapiere, die durch Imprägnation eines säurebeständigen absorbierenden Stoffes mit dem verdünnten oder unverdünnten Folin-Ciocalteu-Reagenz erhalten und getrocknet werden,
eine oder mehrere Einwegpipetten,
Natriumkarbonat-Lösung in entionisiertem Wasser, in einer Tropfflasche enthalten,
**dadurch gekennzeichnet, dass** er ferner umfasst:
eine Referenzfarbkarte, auf der rote, grüne und blaue Bereiche aufgedruckt sind.

14. Der Testsatz nach Anspruch 13, der ferner einen oder mehrere Einweg-Plastikbeutel zum Auspressen von Saft aus Früchten umfasst.

## Revendications

1. Procédé d'analyse pour l'analyse de la concentration totale de phénols et de polyphénols dans des échantillons liquides aqueux, comprenant les étapes consistant à
(a) époser une goutte de l'échantillon sur le papier test préalablement imprégné du réactif de Folin-Ciocalteu formant ainsi une tache test;
(b) ajouter une goutte d'une solution aqueuse alcaline au point test sur le papier test;
(c) placer le papier test sur la carte de couleur de référence avec la tache de test à proximité des zones de référence rouge, verte et bleue imprimées sur la carte de couleur de référence;
(d) prendre une photographie en couleur du point test sur le papier test avec les zones rouges, vertes et bleues sur la carte de couleur de référence;
(e) effectuer une analyse de couleur de la photographie dans le schéma RVB, normalisée en référence aux zones rouge, verte et bleue sur la carte de couleur de référence, et obtenir une mesure de valeur de couleur ;
(f) évaluer la concentration totale de phénols et de polyphénols sur la base des mesures de valeur de couleur rouge, vert et bleu, de préférence vert et bleu.

2. Procédé d'analyse selon la revendication 1, dans lequel après l'étape (a) consistant à déposer une goutte de l'échantillon sur le papier test et avant l'étape (b) consistant à ajouter une solution aqueuse alcaline au point test, les étapes suivantes sont effectuées:
(aa) placer le papier test sur la carte de couleur de référence avec la tache de test à proximité des zones de référence rouge, verte et bleue imprimées sur la carte de couleur de référence;
(ab) prendre une photographie en couleur du point test sur le papier test avec les zones rouges, vertes et bleues sur la carte de couleur de référence;
(ac) effectuer une analyse des couleurs de la photographie dans le schéma RVB normalisé en référence aux zones rouges, vertes et bleues sur la carte de couleurs de référence et déterminer la valeur de couleur bleue de l'échantillon, dans lequel si ladite valeur de couleur bleue n'interfère pas avec la plage de teneur en polyphénols, ladite teneur en polyphénols est estimée sur la base de la valeur de couleur bleue, sinon elle est estimée sur la base de la valeur de couleur verte obtenue en tant que différence des valeurs de couleur verte pour les photographies prises à l'étape (ab) et (d).

3. Procédé d'analyse selon la revendication 1 ou 2, dans lequel l'analyse aux étapes (ac) et (e) est effectuée à l'aide de moyens informatiques capables d'exécuter une application d'analyse de la photographie dans le schéma RVB.

4. Procédé d'analyse selon l'une quelconque des revendications 1 à 3, dans lequel les photographies sont prises et les analyses de couleur desdites photographies sont effectuées en utilisant le même dispositif.

5. Procédé d'analyse selon la revendication 4, dans lequel le dispositif est un smartphone.

6. Procédé d'analyse selon l'une quelconque des revendications 1 à 5, dans lequel l'échantillon est choisi parmi le jus de fruit cru, les boissons contenant du jus, le thé vert, le thé noir, le yerba mate, le café, le vin rouge et la bière.

7. Procédé d'analyse selon la revendication 6, dans lequel l'échantillon est un jus de fruit cru fraîchement pressé.

8. Procédé d'analyse selon l'une quelconque des revendications 1 à 7, dans lequel la photographie de l'étape (d) est prise 1 à 7 minutes après l'exécution de l'étape (b).

9. Procédé d'analyse selon l'une quelconque des revendications 1 à 8, dans lequel la solution alcaline aqueuse de l'étape (b) est une solution de carbonate de sodium.

10. Procédé d'analyse selon l'une quelconque des revendications 1 à 9, dans lequel le papier test est obtenu en imprégnant un matériau absorbant résistant aux acides avec le réactif de Folin-Ciocalteu dilué ou non dilué et séché.

11. Procédé d'analyse selon la revendication 10, dans lequel le matériau absorbant résistant aux acides est choisi dans un groupe comprenant un papier filtre dur, un tissu filtrant ou un tissu non tissé.

12. Procédé d'analyse selon la revendication 10 ou 11, dans lequel le réactif de Folin-Ciocalteu est dilué dans une plage de 10 à 20 fois avec de l'eau désionisée ou une solution de gomme xanthane à 1 %.

13. Kit de test pour la détermination de la concentration totale de phénols et de polyphénols dans des liquides aqueux, comprenant:
un ou plusieurs papier tests obtenus par imprégnation d'un matériau absorbant résistant aux acides avec le réactif de Folin-Ciocalteu dilué ou non dilué et séché;
une ou plusieurs pipettes jetables;
solution de carbonate de sodium dans de l'eau désionisée, contenue dans un flacon compte-goutte
**caractérisé en ce qu'**il comprend en outre
une carte de couleur de référence, avec des zones rouges, vertes et bleues imprimées dessus.

14. Kit de test selon la revendication 13, comprenant en outre un ou plusieurs sacs en plastique jetables pour presser le jus des fruits.
